# EUROPÄISCHE PATENTSCHRIFT

(11) **EP 0 712 429 B1**
(45) Veröffentlichungstag und Bekanntmachung des Hinweises auf die Patenterteilung: **18.11.1998**
(21) Anmeldenummer: 94926165.5
(22) Anmeldetag: 09.08.1994
(51) Int. Cl.: C09B 29/36, C07D 471/04, B41M 5/38

(54) **PHENYLAZOTRIAZOLOPYRIDINFARBSTOFFE**
PHENYLAZOTRIAZOLOPYRIDINE DYES
COLORANTS DE LA PHENYLAZOTRIAZOLOPYRIDINE

(30) Priorität: 13.08.1993 DE 4327222
(43) Veröffentlichungstag der Anmeldung: 22.05.1996
(73) Patentinhaber: BASF AKTIENGESELLSCHAFT, 67056 Ludwigshafen (DE)
(72) Erfinder: SCHEFCZIK, Ernst, D-67069 Ludwigshafen (DE); SENS, Ruediger, D-68165 Mannheim (DE); ETZBACH, Karl-Heinz, D-67227 Frankenthal (DE); REICHELT, Helmut, D-67435 Neustadt (DE); GRUND, Clemens, D-68199 Mannheim (DE)
(86) Internationale Anmeldenummer: EP9402634
(87) Internationale Veröffentlichungsnummer: WO9505420

(56) Entgegenhaltungen:
- EP-A- 0 413 226
- DE-A- 4 020 768

## Beschreibung

Die vorliegende Erfindung betrifft neue Azofarbstoffe der Formel I 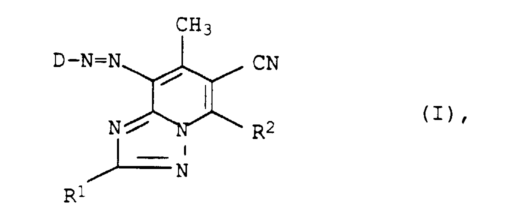 in der
- R¹: C₁-C₂₀-Alkyl, das gegebenenfalls substituiert ist und durch 1 bis 4 Sauerstoffatome in Etherfunktion unterbrochen sein kann, gegebenenfalls substituiertes Phenyl, Mercapto oder gegebenenfalls substituiertes Alkylthio,
- R²: Hydroxy oder Mercapto und
- D: den Rest einer Diazokomponente, die ausgewählt ist aus der Gruppe, bestehend aus den Resten der Formeln 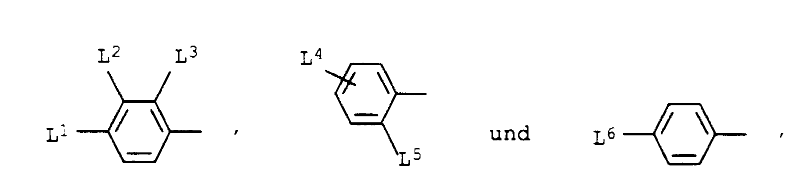 worin L¹ für C₁-C₄-Alkyl, C₁-C₄-Alkoxy, das gegebenenfalls durch C₁-C₄-Alkoxy substituiert ist, C₁-C₄-Alkanoylamino, C₁-C₄-Alkylsulfonylamino oder einen Rest der Formel NY¹Y², worin Y¹ und Y² unabhängig voneinander jeweils die Bedeutung von Wasserstoff oder C₁-C₄-Alkyl oder zusammen mit dem sie verbindenden Stickstoffatom die Bedeutung eines 5- oder 6-gliedrigen gesättigten heterocyclischen Restes besitzen, einer der beiden Reste L² und L³ für Wasserstoff und der andere für C₁-C₄-Alkyl, C₁-C₄-Alkoxy, das gegebenenfalls durch C₁-C₄-Alkoxy substituiert ist, C₁-C₄-Alkanoylamino, C₁-C₄-Alkylsulfonylamino oder einen Rest der Formel NY¹Y², worin Y¹ und Y² jeweils die obengenannte Bedeutung besitzen, L⁴ für Wasserstoff oder C₁-C₄-Alkyl, L⁵ für C₁-C₄-Alkoxy, das gegebenenfalls durch C₁-C₄-Alkoxy substituiert ist, oder C₁-C₄-Alkylthio und L⁶ für C₈-C₁₃-Alkyl, das durch 1 bis 4 Sauerstoffatome in Etherfunktion unterbrochen sein kann, oder Phenoxy stehen, bedeuten,
deren Verwendung zum Färben oder Bedrucken von textilen Materialien sowie neue Mercaptotriazolopyridine.

Aus der DE-A-4 020 768 sind bereits Triazolopyridinazofarbstoffe mit Diazokomponenten aus der Anilinreihe bekannt. Es hat sich jedoch gezeigt, daß die dort beschriebenen Farbstoffe noch anwendungstechnische Mängel aufweisen.

Aufgabe der vorliegenden Erfindung war es daher, neue Azofarbstoffe mit einer Kupplungskomponente aus der Triazolopyridinreihe und einer Diazokomponente aus der Anilinreihe bereitzustellen, die über eine hohe Thermofixierechtheit, hohe Brillanz sowie über hohe Farbstärke verfügen.

Demgemäß wurden die eingangs näher bezeichneten Azofarbstoffe der Formel I gefunden.

Die Farbstoffe der Formel I können in mehreren tautomeren Formen auftreten, die alle von den Patentansprüchen umfaßt werden. Beispielsweise können die Farbstoffe in folgenden tautomeren Formen auftreten: 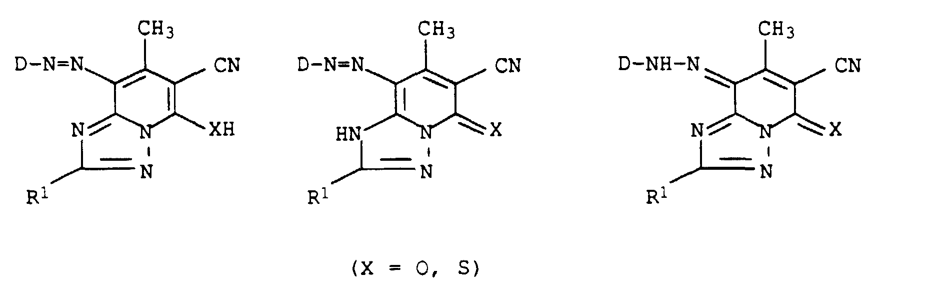 Wenn R¹ in Formel I einen substituierten C₁-C₂₀-Alkylrest bedeutet, so können als Substituenten z.B. Phenyl, Phenoxy, Carboxyl oder C₁-C₂₀-Alkoxycarbonyl, dessen Alkylkette durch 1 bis 4 Sauerstoffatome in Etherfunktion unterbrochen sein kann und gegebenenfalls durch Phenyl oder Phenoxy substituiert ist, in Betracht kommen. Die Alkylreste weisen dann in der Regel 1 oder 2 Substituenten auf.

Wenn in Formel I Alkylreste auftreten, die durch Sauerstoffatome in Etherfunktion unterbrochen sind, so sind solche Alkylreste bevorzugt, die durch 1 oder 2 Sauerstoffatome in Etherfunktion unterbrochen sind.

Wenn in Formel I substituierte Phenylreste auftreten, so können, sofern nicht anders vermerkt, als Substituenten z.B. C₁-C₄-Alkyl, C₁-C₄-Alkoxy, Halogen, dabei insbesondere Chlor oder Brom, Cyano, Nitro oder Carboxyl in Betracht kommen. Die Phenylreste weisen dann in der Regel 1 bis 3 Substituenten auf.

Wenn die Reste Y¹ und Y² zusammen mit dem sie verbindenden Stickstoffatom einen 5- oder 6-gliedrigen gesättigten heterocyclischen Rest bedeuten, so können dafür z.B. Pyrrolidinyl, Piperidinyl, Morpholinyl, Piperazinyl oder N-(C₁-C₄-Alkyl)piperazinyl in Betracht kommen.

Reste R¹, L¹, L², L³, L⁴, Y¹ und Y² sind z.B. Methyl, Ethyl, Propyl, Isopropyl, Butyl, Isobutyl oder sec-Butyl.

Reste R¹ sind weiterhin z.B. Pentyl, Isopentyl, Neopentyl, tert-Pentyl, Hexyl, Heptyl, 1-Ethylpentyl, Octyl, Isooctyl, 2-Ethylhexyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl, Eicosyl (Die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmanns Enzyklopädie der technischen Chemie, 4. Auflage, Band 7, Seiten 215 bis 217, sowie Band 11, Seiten 435 und 436.), 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- oder 4-Butoxybutyl, 4,8-Dioxadecyl, 3,6,9-Trioxadecyl, 3,6,9-Trioxaundecyl, 3,6,9,12-Tetraoxatridecyl, 3,6,9,12-Tetraoxatetradecyl, 2-Carboxyethyl, 2-Methoxycarbonylethyl, Benzyl, 1- oder 2-Phenylethyl, 2-, 3- oder 4-Methylbenzyl, 2-, 3- oder 4-Methoxybenzyl, 2-, 3- oder 4-Chlorbenzyl, 2-, 3- oder 4-Nitrobenzyl, 3-Benzyloxypropyl, Phenoxymethyl, 6-Phenoxy-4-oxahexyl, 8-Phenoxy-4-oxaoctyl, 2-, 3- oder 4-Methylphenyl, 2-, 3- oder 4-Methoxyphenyl, 2-, 3- oder 4-Chlorphenyl, 2-, 3- oder 4-Nitrophenyl, 2-, 3- oder 4-Carboxyphenyl, Methylthio, Ethylthio, Propylthio, Isopropylthio, Butylthio, Isobutylthio, sec-Butylthio, Pentylthio, Isopentylthio, Neopentylthio, tert-Pentylthio, Hexylthio, Heptylthio, 1-Ethylpentylthio, Octylthio, Isooctylthio, 2-Ethylhexylthio, Nonylthio, Isononylthio, Decylthio, Isodecylthio, Undecylthio, Dodecylthio, Tridecylthio, Isotridecylthio, Tetradecylthio, Pentadecylthio, Hexadecylthio, Heptadecylthio, Octadecylthio, Nonadeylthio oder Eicosylthio.

Reste L¹, L², L³ und L⁵ sind weiterhin z.B. Methoxy, Ethoxy, Propoxy, Isopropoxy, Butoxy, Isobutoxy, sec-Butoxy, 2-Methoxyethyl, 2-Ethoxyethyl, 2-Propoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Propoxybutyl oder 2- oder 4-Butoxybutyl.

Reste L⁵ sind weiterhin z.B. Methylthio, Ethylthio, Propylthio, Isopropylthio oder Butylthio.

Reste L¹, L² und L³ sind weiterhin z.B. Formylamino, Acetylamino, Propionylamino, Butyrylamino, Isobutyrylamino, Methylsulfonylamino, Ethylsulfonylamino, Propylsulfonylamino, Isopropylsulfonylamino oder Butylsulfonylamino.

Reste L⁶ sind z.B. Isooctyl, 2-Ethylhexyl, Nonyl, Isononyl, Decyl, Isodecyl, Undecyl, Dodecyl, Tridecyl, Isotridecyl, 2- oder 4-Butoxybutyl, 4,8-Dioxadecyl, 3,6,9-Trioxaundecyl, 3,6,9,12-Tetraoxatridecyl oder 3,6,9,12-Tetraoxatetradecyl.

Bevorzugt sind Azofarbstoffe der Formel I, in der R² Hydroxy bedeutet.

Weiterhin bevorzugt sind Azofarbstoffe der Formel I, in der R¹ C₁-C₁₀-Alkyl, insbesondere C₁-C₈-Alkyl bedeutet.

Wenn D in Formel I für einen Rest der Formel 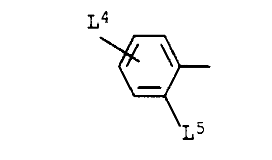 steht, so sind solche Farbstoffe bevorzugt, in denen die Substituenten L⁴ und L⁵ folgende Anordnung haben 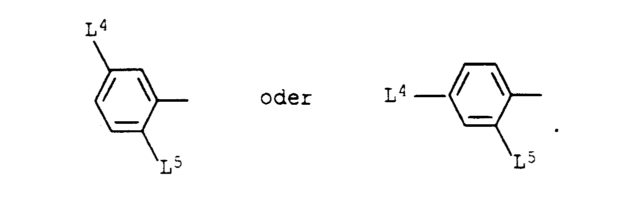 Die neuen Azofarbstoffe der Formel I können nach an sich bekannten Methoden hergestellt werden. Beispielsweise kann man auf an sich bekanntem Weg eine Diazokomponente der Formel III

D-NH₂ (III),

in der D die obengenannte Bedeutung besitzt, diazotieren und mit einem Triazolopyridin der Formel IV 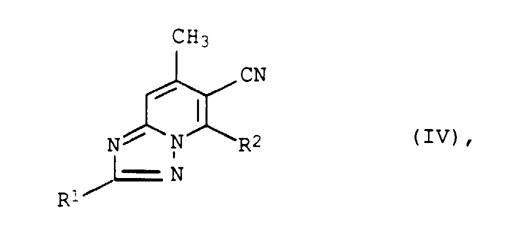 in der R¹ und R² jeweils die obengenannte Bedeutung besitzen, kuppeln.

Bei den Triazolopyridinen der Formel IV (R²= Hydroxy) handelt es sich um an sich bekannte Verbindungen. Sie sind z.B. in der US-A-5 101 028 beschrieben.

Ein weiterer Gegenstand der vorliegenden Erfindung sind Mercaptotriazolopyridine der Formel II 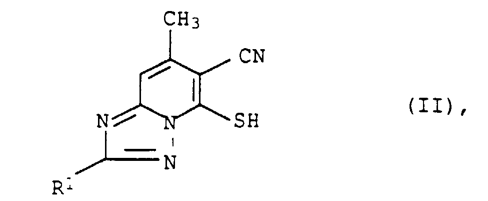 in der R¹ C₁-C₂₀-Alkyl, das gegebenenfalls substituiert ist und durch 1 bis 4 Sauerstoffatome in Etherfunktion unterbrochen sein kann, oder gegebenenfalls substituiertes Phenyl bedeuten.

Bevorzugt sind Mercaptotriazolopyridine der Formel II, in der R¹ C₁-C₁₀-Alkyl, insbesondere C₁-C₈-Alkyl bedeutet.

Die neuen Mercaptotriazolopyridine der Formel II können z.B. erhalten werden, indem man Halogentriazolopyridine der Formel V 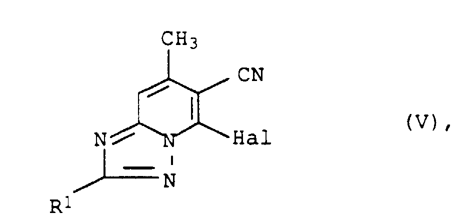 in der Hal Chlor oder Brom bedeutet und R¹ die obengenannte Bedeutung besitzt, mit Schwefelwasserstoff behandelt. Die Halogentriazolopyridine der Formel V sind ebenfalls aus der US-A-5 101 028 bekannt.

Die erfindungsgemäßen Mercaptotriazolopyridine der Formel II sind wertvolle Zwischenprodukte für die Herstellung von Azofarbstoffen, insbesondere von solchen der Formel I.

Die erfindungsgemäßen Azofarbstoffe der Formel I eignen sich in vorteilhafter Weise zum Färben oder Bedrucken von textilen Materialien. Dies sind beispielsweise Fasern oder Gewebe aus Celluloseestern oder Polyestern, aber auch aus Polyamiden oder Mischgewebe aus Polyestern und Cellulosefasern. Man erhält dabei Färbungen oder Drucke mit hoher Thermofixierechtheit und hoher Brillanz. Die neuen Farbstoffe weisen auch eine hohe Farbstärke auf.

Um einen günstigen Farbaufbau zu erreichen, kann es in manchen Fällen von Vorteil sein, Mischungen der Farbstoffe der Formel I untereinander zum Färben zu verwenden.

Die erfindungsgemäßen Azofarbstoffe eignen sich weiterhin vorteilhaft für die thermische Übertragung von einem Träger auf ein mit Kunststoff beschichtetes Papier mittels einer Energiequelle (siehe z.B. US-A-5 079 365).

Die folgenden Beispiele sollen die Erfindung näher erläutern.

### Beispiel 1

### a) Diazotierung

4,00 g (0,02 mol) 2-Butoxy-5-methylanilin wurden in 80 ml Eisessig/Propionsäure (3:1 v/v) und 16 ml 85 gew.-%iger Schwefelsäure gelöst. Bei maximal -5°C bis 0°C wurden 10,4 g Nitrosylschwefelsäure (ca. 42 Gew.-% N₂O₃) zugetropft. Es wurde 2 h bei maximal 0°C nachgerührt.

### b) Kupplung

5,40 g (0,022 mol) 6-Cyano-2-(1'-ethylpropyl)-7-hydroxy-5-methyl[1,2,4]triazolo[1,5-a]pyridin wurden in 90 ml N-Methylpyrrolidon gelöst und mit 100 ml Wasser, 0,5 g Amidosulfonsäure und ca. 400 g Eis versetzt. Die unter a) beschriebene Diazoniumsalzlösung wurde portionsweise zugegeben, wobei mit 25 gew.-%iger Natronlauge der pH-Wert auf 6,5 bis 7,0 eingestellt wurde. Anschließend rührte man 3 h bei 0 bis 5°C nach. Nach beendeter Kupplung wurde der Farbstoff bei 60°C abgesaugt, neutral gewaschen und bei 60°C unter vermindertem Druck getrocknet. Man erhielt 5,20 g (64 % d.Th.) des Farbstoffs der Formel 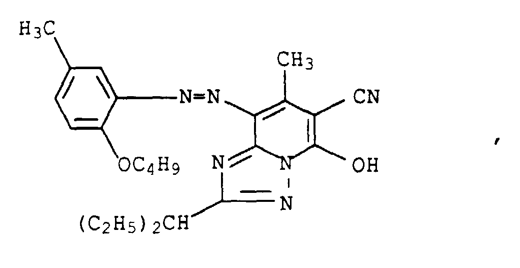 (Fp.: 225 bis 228°C; λₘₐₓ (CH₂Cl₂): 508 nm) der Polyester in roter Nuance färbt.

### Beispiel 2

### a) Diazotierung

3,90 g (0,025 mol) 2,4-Dimethoxyanilin wurden bei 0 bis 5°C in eine Mischung aus 20 ml Wasser, 7,5 g 35 gew.-%ige Salzsäure und 15 g Eis eingetragen. Bei 0 bis 5°C wurden 8 g 23 gew.-%ige wäßrige Natriumnitritlösung zugetropft und 3 h bei 0 bis 5°C gerührt.

### b) Kupplung

6,40 g (0,026 mol) 6-Cyano-2-(1'-ethylpropyl) -7-hydroxy-5-methyl[1,2,4]triazolo[1,5-a]pyridin wurden in einer Mischung aus 20 ml N-Methylpyrrolidon, 30 g Eis, 4 g 50 gew.-%ige Natronlauge und 2 g Natriumhydrogencarbonat gelöst. Nach Zugabe von 100 g Eis wurde die unter a) beschriebene Diazoniumsalzlösung innerhalb von 10 min zugegeben und 5 h bei 5°C nachgerührt. Nach beendeter Kupplung wurde der Farbstoff bei 60°C abgesaugt, mit 60°C warmem Wasser neutral gewaschen und bei 60°C unter vermindertem Druck getrocknet. Man erhielt 5 g (49 % d.Th.) des Farbstoffs der Formel 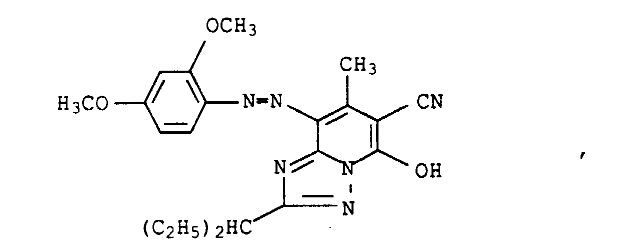 (Fp.: > 230°C; λₘₐₓ (CH₂Cl₂) : 514 nm) der Polyester in roter Nuance färbt.

In analoger Weise können die in der folgenden Tabelle aufgeführten Farbstoffe erhalten werden.

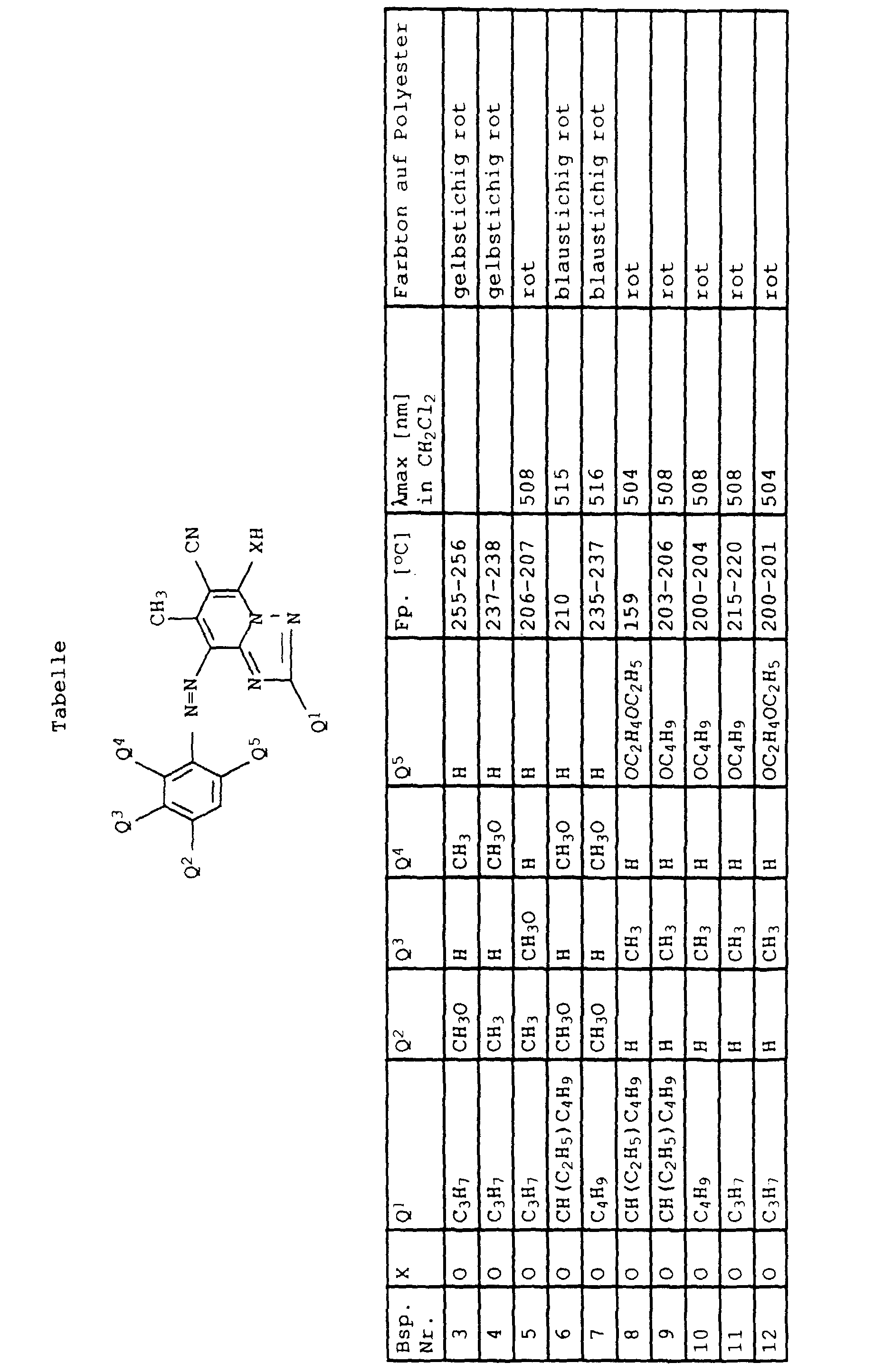

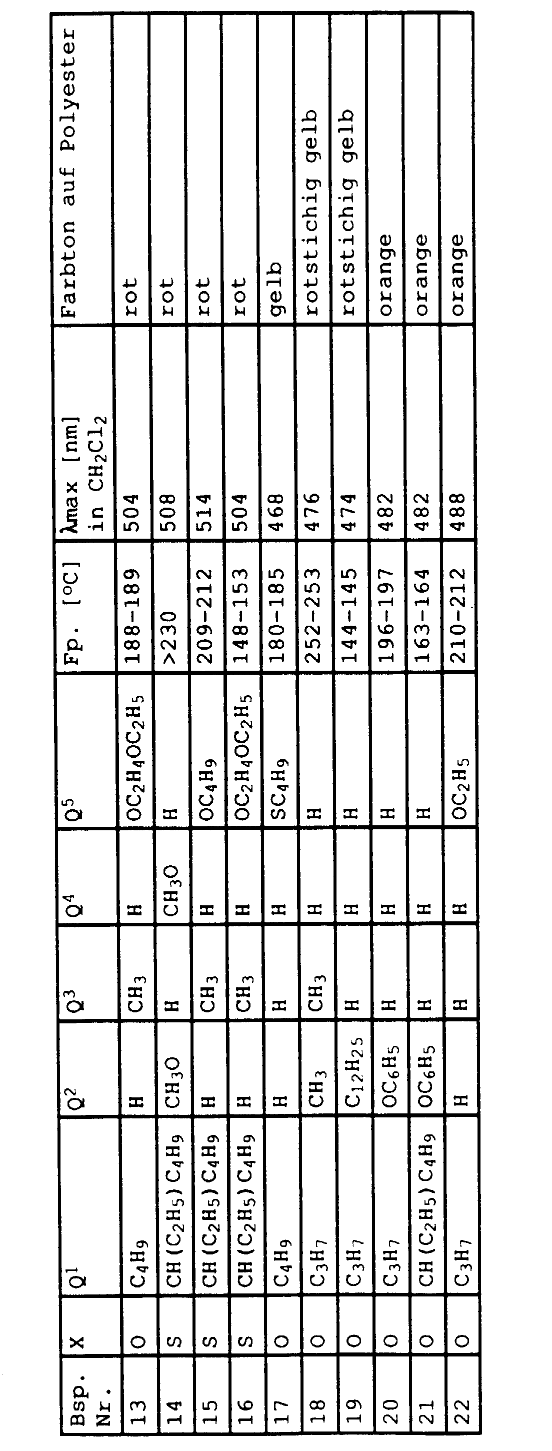

### Beispiel 23

Zu einem Gemisch aus 300 ml 1-Methoxypropan-2-ol und 25 g Triethylamin wurde 1 h lang unter Eiskühlung gasförmiger Schwefelwasserstoff gegeben. Danach wurden 58 g der Verbindung der Formel 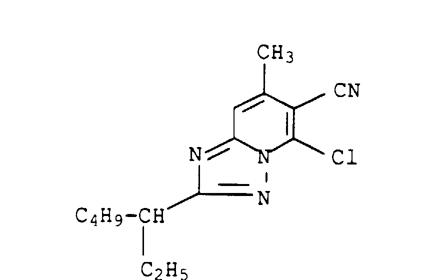 zugegeben und für weitere 4 h gasförmiger Schwefelwasserstoff eingeleitet. Das resultierende Reaktionsgemisch wurde auf 18 gew.-%ige Salzsäure gegossen, wobei sich ein Niederschlag bildete. Dieser wurde abgesaugt, mit Wasser gewaschen und bei 80°C unter vermindertem Druck getrocknet. Man erhielt 51 g der Verbindung der Formel 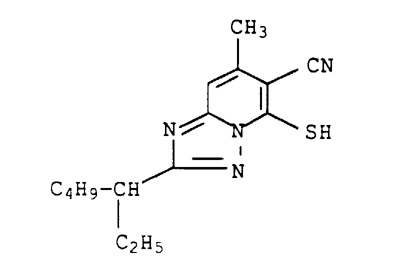 Schmelzpunkt (aus N,N-Dimethylformamid/Essigsäure): 270 bis 271°C

| Analyse: C₁₅H₂₀N₄S (288) | | | | |
|---|---|---|---|---|
| ber. | C 62,5 | H 7,0 | N 19,4 | S 11,1 |
| gef. | C 62,5 | H 7,0 | N 19,4 | S 10,8 |

## Patentansprüche

1. Azofarbstoffe der Formel I 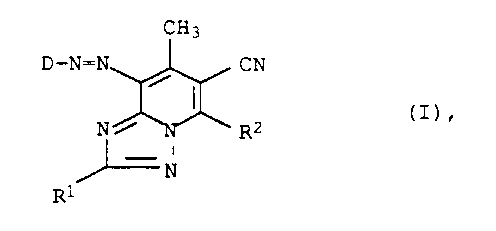 in der
R¹ C₁-C₂₀-Alkyl, das gegebenenfalls substituiert ist und durch 1 bis 4 Sauerstoffatome in Etherfunktion unterbrochen sein kann, gegebenenfalls substituiertes Phenyl, Mercapto oder gegebenenfalls substituiertes Alkylthio,
R² Hydroxy oder Mercapto und
D den Rest einer Diazokomponente, die ausgewählt ist aus der Gruppe, bestehend aus den Resten der Formeln 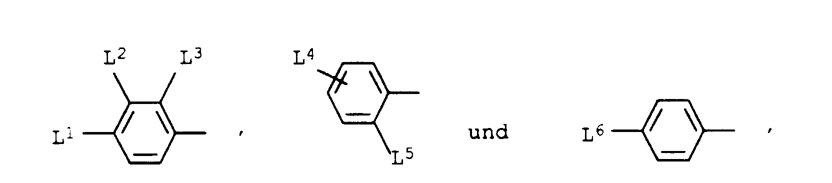 worin L¹ für C₁-C₄-Alkyl, C₁-C₄-Alkoxy, das gegebenenfalls durch C₁-C₄-Alkoxy substituiert ist, C₁-C₄-Alkanoylamino, C₁-C₄-Alkylsulfonylamino oder einen Rest der Formel NY¹Y², worin Y¹ und Y² unabhängig voneinander jeweils die Bedeutung von Wasserstoff oder C₁-C₄-Alkyl oder zusammen mit dem sie verbindenden Stickstoffatom die Bedeutung eines 5- oder 6-gliedrigen gesättigten heterocyclischen Restes besitzen, einer der beiden Reste L² und L³ für Wasserstoff und der andere für C₁-C₄-Alkyl, C₁-C₄-Alkoxy, das gegebenenfalls durch C₁-C₄-Alkoxy substituiert ist, C₁-C₄-Alkanoylamino, C₁-C₄-Alkylsulfonylamino oder einen Rest der Formel NY¹Y², worin Y¹ und Y² jeweils die obengenannte Bedeutung besitzen, L⁴ für Wasserstoff oder C₁-C₄-Alkyl, L⁵ für C₁-C₄-Alkoxy, das gegebenenfalls durch C₁-C₄-Alkoxy substituiert ist, oder C₁-C₄-Alkylthio und L⁶ für C₈-C₁₃-Alkyl, das durch 1 bis 4 Sauerstoffatome in Etherfunktion unterbrochen sein kann, oder Phenoxy stehen, bedeuten.

2. Azofarbstoffe nach Anspruch 1, dadurch gekennzeichnet, daß R² Hydroxy bedeutet.

3. Azofarbstoffe nach Anspruch 1, dadurch gekennzeichnet, daß R¹ C₁-C₁₀-Alkyl bedeutet.

4. Verwendung der Azofarbstoffe gemäß Anspruch 1 zum Färben oder Bedrucken von textilen Materialien.

5. Mercaptotriazolopyridine der Formel II 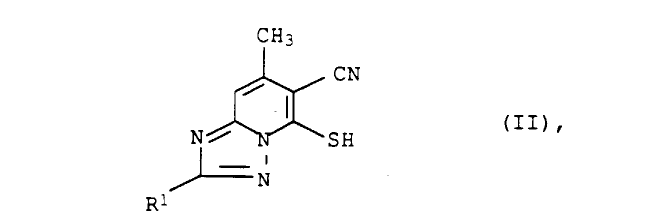 in der
R¹ C₁-C₂₀-Alkyl, das gegebenenfalls substituiert ist und durch 1 bis 4 Sauerstoffatome in Etherfunktion unterbrochen sein kann, oder gegebenenfalls substituiertes Phenyl bedeutet.

## Claims

1. An azo dyestuff of the formula I 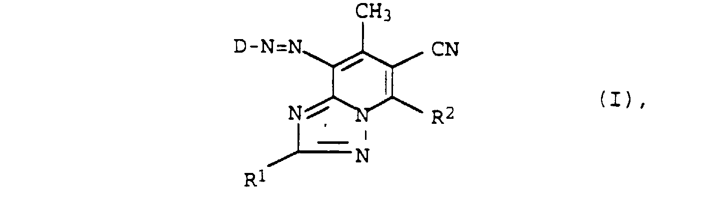 where
R¹ is C₁-C₂₀-alkyl which is unsubstituted or substituted and can be interrupted by 1 to 4 ether oxygen atoms, or is unsubstituted or substituted phenyl, or mercapto or unsubstituted or substituted alkylthio,
R² is hydroxyl or mercapto and
D is the radical of a diazo component which is selected from the group consisting of the radicals of the formulae 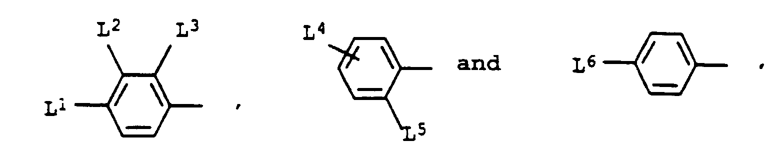 where L¹ is C₁-C₄-alkyl, C₁-C₄-alkoxy which is unsubstituted or substituted by C₁-C₄-alkoxy, or is C₁-C₄-alkanoylamino, C₁-C₄-alkylsulfonylamino or a radical of the formula NY¹Y², where Y¹ and Y² independently of one another in each case are hydrogen or C₁-C₄-alkyl or, together with the nitrogen bonding them, are a 5- or 6-membered saturated heterocyclic radical, one of the two radicals L² and L³ is hydrogen and the other is C₁-C₄-alkyl, C₁-C₄-alkoxy which is unsubstituted or substituted by C₁-C₄-alkoxy, or is C₁-C₄-alkanoylamino, C₁-C₄-alkylsulfonylamino or a radical of the formula NY¹Y², in which Y¹ and Y² in each case have the abovementioned meanings, L⁴ is hydrogen or C₁-C₄-alkyl, L⁵ is C₁-C₄-alkoxy which is unsubstituted or substituted by C₁-C₄-alkoxy, or is C₁-C₄-alkylthio and L⁶ is C₈-C₁₃-alkyl which can be interrupted by 1 to 4 ether oxygen atoms, or is phenoxy.

2. An azo dyestuff as claimed in claim 1, wherein R² is hydroxyl.

3. An azo dyestuff as claimed in claim 1, wherein R¹ is C₁-C₁₀-alkyl.

4. The use of the azo dyestuffs as claimed in claim 1 for dyeing or printing textile materials.

5. A mercaptotriazolopyridine of the formula II 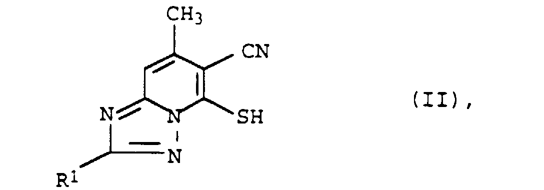 where
R¹ is C₁-C₂₀-alkyl which is unsubstituted or substituted and can be interrupted by 1 to 4 ether oxygen atoms, or is unsubstituted or substituted phenyl.

## Revendications

1. Colorants azoïques de formule I 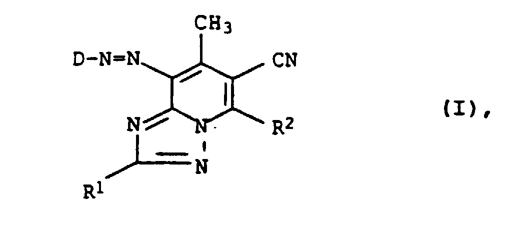 dans laquelle
R¹ représente un groupement alkyle en C₁-C₂₀ éventuellement substitué et pouvant être interrompu par 1 à 4 atomes d'oxygène en fonction éther, un groupement phényle éventuellement substitué, un groupement mercapto ou un groupement alkylthio éventuellement substitué,
R² représente un groupement hydroxy ou mercapto et
D le reste d'un composé diazoïque choisi dans le groupe constitué par les restes de formules 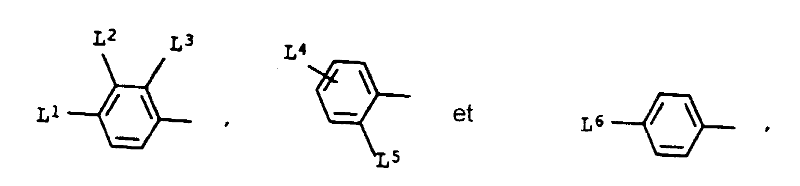 dans lesquelles L¹ représente un groupement alkyle en C₁-C₄, alcoxy en C₁-C₄ éventuellement substitué par des groupements alcoxy en C₁-C₄, un groupement alcanoylamino en C₁-C₄, un groupement alkylsulfonylamino en C₁-C₄ ou un reste de formule NY¹Y², où Y¹ et Y² représentent chacun indépendamment l'un de l'autre un atome d'hydrogène ou un groupement alkyle en C₁-C₄ ou bien, avec l'atome d'azote qui les relie, un reste hétérocyclique saturé à 5 ou 6 chaînons, l'un des restes L² et L³ représente un atome d'hydrogène et l'autre un groupement alkyle en C₁-C₄, alcoxy en C₁-C₄, éventuellement substitué par un groupement alcoxy en C₁-C₄, un groupement alcanoylamino en C₁-C₄, alkylsulfonylamino en C₁-C₄ ou un reste de formule NY¹Y², où Y¹ et Y² prennent chacun la signification susmentionnée, L⁴ représente un atome d'hydrogène ou un groupement alkyle en C₁-C₄, L⁵ représente un groupement alcoxy en C₁-C₄ éventuellement substitué par des groupements alcoxy en C₁-C₄, ou un groupement alkylthio en C₁-C₄ et L⁶ représente un groupement alkyle en C₈-C₁₃ pouvant être interrompu par 1 à 4 atomes d'oxygène en fonction éther, ou un groupement phénoxy.

2. Colorants azoïques selon la revendication 1, caractérisé en ce que R² représente un groupement hydroxy.

3. Colorants azoïques selon la revendication 1, caractérisé en ce que R¹ représente un groupement alkyle en C₁-C₁₀.

4. Utilisation de colorants azoïques selon la revendication 1 pour la coloration ou l'impression de matériaux textiles.

5. Mercaptotriazolopyridines de formule II 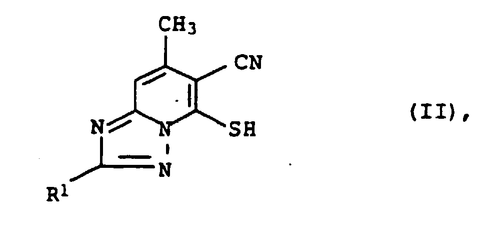 dans laquelle
R¹ représente un groupement alkyle en C₁-C₂₀ éventuellement substitué et pouvant être interrompu par 1 à 4 atomes d'oxygène en fonction éther, ou bien un groupement phényle éventuellement substitué.
